# EUROPÄISCHE PATENTSCHRIFT

(11) **EP 1 371 358 B1**
(45) Veröffentlichungstag und Bekanntmachung des Hinweises auf die Patenterteilung: **15.08.2007**
(21) Anmeldenummer: 03007934.7
(22) Anmeldetag: 08.04.2003
(51) Int. Cl.: A61K 8/49, A61K 8/81, A61K 8/87, A61K 8/88, A61K 8/89, A61Q 17/04

(54) **Kosmetische und dermatologische Lichtschutzzubereitungen enthaltend UV-Absorber und synthetische organische Polymere**
Cosmetic and dermatological sunscreening compositions containing UV-absorbers and synthetic organic polymers
Compositions photoprotectrices cosmétiques et dermatologiques contenant des agents absorbant l'UV et des polymères organiques synthétiques

(30) Priorität: 13.06.2002 DE 10226352
(43) Veröffentlichungstag der Anmeldung: 17.12.2003
(73) Patentinhaber: Beiersdorf AG, 20253 Hamburg (DE)
(72) Erfinder: Lanzendörfer, Ghita, Dr., 22087 Hamburg (DE); Schulz, Jens, Dr., 12169 Berlin (DE); Kruse, Uta, 20149 Hamburg (DE); Knüppel, Anja, 22257 Hamburg (DE)
(74) Vertreter: UEXKÜLL & STOLBERG

(56) Entgegenhaltungen:
- EP-A- 0 893 119
- EP-A- 1 077 058
- FR-A- 2 816 836
- ANON.: "The use of mixtures of the UVA-absorber methylene bis-benzotriazolyl tetramethylbutylphenol with UVB-absorber in sunscreen formulations " RESEARCH DISCLOSURE, Nr. 449, 2001, Seiten 1478-1484, XP007128884 GB

## Beschreibung

Die vorliegende Erfindung betrifft kosmetische und dermatologische Zubereitungen zum Schutz von Haut und Haar gegen UV-Strahlung sowie deren Verwendung zu dem genannten Zweck, insbesondere im Bereich der dekorativen Kosmetik.

Die schädigende Wirkung des ultravioletten Teils der Sonnenstrahlung ist bekannt. Die UV-Strahlung im Bereich zwischen 290 nm und 320 nm, dem sogenannten UV-B-Bereich kann zu einem Erythem, einem einfachen Sonnenbrand oder sogar mehr oder weniger starken Verbrennungen als akuten Erscheinungsformen führen. Als ein Maximum der Erythemwirksamkeit des Sonnenlichtes wird der engere Bereich um 308 nm angegeben.

Man hat lange Zeit fälschlicherweise angenommen, dass die längerwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, dass UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u. a. erwiesen, dass selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Es ist daher von grundsätzlicher Wichtigkeit, dass kosmetische und dermatologische Lichtschutzzubereitungen sowohl gegen UV-B- als auch gegen UV-A-Strahlung ausreichenden Schutz bieten. Für diese Zwecke sind im Stand der Technik schon eine Vielzahl von kosmetischen Zubereitungen entwickelt worden. Allerdings ist deren Wirkungsspektrum weiter verbesserbar. Insbesondere weisen Zubereitungen, die Kombinationen von UV-Filtersubstanzen oder anderen Wirkstoffen enthalten, bisweilen in Bezug auf die Einsatzkonzentrationen formulierungstechnische Schwierigkeiten auf, die das Erreichen guter UV-Schutzleistung erschweren.

Die EP-A-0 893 119 offenbart ein Sonnenschutzmittel, das für pharmazeutische oder kosmetische Anwendungen geeignet ist, das einen organischen UV-Absorber, einen öllöslichen UV-Absorber und/oder ein anorganisches Mikropigment, und ein polymeres Hohlkugel-Additiv und/oder ein Polymer ausgewählt aus Xanthan und/oder Polyvinylpyrrolidon enthält.

Die EP-A-1 077 058 offenbart kosmetische oder dermatologische Zubereitungen, die feindisperse Systeme vom Typ Wasser-in-Öl darstellen, und eine Ölphase, eine Wasserphase, eine Kombination von mindestens einem modifizierten Schichtsilikat und Bornitrid sowie ein oder mehrere Emulgatoren und gegebenenfalls weitere Hilfsstoffe enthalten.

Die FR-A-2 816 836 betrifft eine kosmetische oder dermatologische Zusammensetzung zur Selbstbräunung der Haut, die UV-Strahlung filternde Substanzen beinhaltet.

Die Offenbarung RD 449030 nennt verschiedene UVA- und UVB-Absorber, die zur Verwendung in Sonnenschutzformulierungen geeignet sind.

Neben den oben bereits beschriebenen Wirkungen der UV-Strahlen kommt es in der Nachreaktion der Haut ferner zu einer verminderten Sebumproduktion und einem Austrocknen der Haut. Zur Linderung und zur Pflege dieser Phänomene kennt der Stand der Technik Produkte, welche nach dem Sonnenbaden angewendet werden und üblicherweise spezielle Wirkstoffe enthalten, wie beispielsweise
■ Rückfettungs- und Feuchthaltemittel,
■ entzündungslindernde und kühlende Stoffe,
■ lokal anaestesierende Stoffe und/oder
■ desinfizierende Stoffe, um mögliche Hautinfektionen zu verhindern.

Diese sogenannten Aftersun- oder Après-soleil-Präparate sind dazu bestimmt, die Haut nach dem Sonnenbad zu kühlen und ihr Feuchthaltevermögen zu verbessern, wobei die Vermittlung des Kühleffektes eine zentrale Rolle spielt. Allerdings mangelt es dem Stand der Technik an Produkten, welche die Haut bereits während der UV-Einstrahlung vor dem Austrocknen schützen und sie ausreichend pflegen.

Ein weiterer Nachteil des Standes der Technik ist, dass übliche Lichtschutzformulierungen einen meist klebrigen Film auf der Haut hinterlassen. Dies hat bei der Anwendung solcher Produkte z.B. in Form von Sonnenschutzmitteln an einem Sandstrand zur Folge, dass der Sand am Körper haften bleibt, was vom Anwender als unangenehm empfunden wird und im schlimmsten Fall dazu führen kann, dass das Sonnenschutzmittel zu wenig oder gar nicht mehr verwendet wird. Da am Meer meist ein mehr oder weniger starker Wind herrscht, tritt dieser Nachteil in der Regel selbst dann auf, wenn der Körper gar nicht direkt mit dem Sand in Berührung kommt - beispielsweise beim Sonnenbaden auf einem Liegestuhl - da auch der im Wind herumwirbelnde Sandstaub auf den eingecremten Hautpartien haften bleibt.

Aufgabe der vorliegenden Erfindung ist es, Lichtschutzzubereitungen bereitzustellen, die einen hohen Schutz gegen die oben genannten verschiedenen nachteiligen Wirkungen der Strahlung im UV-A- und UV-B-Bereich, insbesondere Sonnenstrahlung, bieten und gleichzeitig verbesserte sensorische Eigenschaften aufweisen. Die erfindungsgemäßen Zubereitungen sollen ferner gute hautpflegende Eigenschaften, insbesondere Wirksamkeit gegen Hautalterungserscheinungen (z.B. Faltenbildung, Elastizitätsverlust), aufweisen sowie anwendungsfreundlich sein, z.B. sandabweisende Wirkung aufweisen.

Erfindungsgemäß wird eine lichtschutzwirksame kosmetische Zubereitung bereitgestellt, die dadurch gekennzeichnet ist, dass sie
(a) mindestens 2 UV-Filtersubstanzen enthält, wobei die erste UV-Filtersubstanz 2,4-Bis-{[4-(2-ethyl-hexyloxy)-2-hydroxy]-phenyl}-6-(4-methoxyphenyl)-1,3,5-triazin und die zweites UV-Filtersubstanz in Komponente (a) Phenylen-1,4-bis-(2-benzimidazyl)-3,3'-5,5'-tetrasulfonsäure-bis-natriumsalz ist, und
(b) mindestens einen Füllstoff, ausgewählt aus synthetischen organischen Polymeren
enthält.

Gegenstand der Erfindung ist ferner eine dermatologische Zubereitung mit Bestandteilen wie zuvor für die kosmetische Zubereitung definiert.

Gegenstand der Erfindung ist ferner die Verwendung der erfindungsgemäßen vorstehend definierten Zubereitungen zum Schutz von Haut und Haar gegen die schädlichen Wirkungen von UV-Strahlung, insbesondere UV-A- und UV-B-Strahlung, insbesondere Sonnenstrahlung.

Bevorzugte Ausführungsformen der vorliegenden Erfindung ergeben sich aus den abhängigen Patentansprüchen.

Synthetisches organisches Polymer im Sinne der vorliegenden Erfindung meint ein Polymer, dass durch Polymerisation synthetisch aus Monomeren erhalten wurde, im Gegensatz zu natürlichen Polymeren wie etwa Stärke oder nur leicht derivatisierten natürlichen Polymeren wie etwa Distarch Phosphate (INCI-Bezeichnung), ein Stärke-Derivat.

Die erfindungsgemäßen Zubereitungen stellen in jeglicher Hinsicht überaus befriedigende Präparate dar, welche im Hinblick auf die weiteren Bestandteile nicht auf eine spezielle Auswahl dieser weiteren Bestandteile begrenzt sind. Dementsprechend eignen sie sich ganz besonders, um als Grundlage für Zubereitungsformen mit vielfältigen Anwendungszwecken zu dienen. Die erfindungsgemäßen Zubereitungen zeigen überraschenderweise synergistisch verbesserte sensorische und kosmetische Eigenschaften, wie beispielsweise die gleichmäßige Verteilbarkeit auf der Haut oder das Einzugsvermögen in die Haut, und zeichnen sich gleichzeitig durch eine hohe Lichtschutzeffektivität bei gleichzeitig hervorragenden Hautpflegedaten aus.

Ein Maß für die UV-Schutzleistung stellt im Sinne der vorliegenden Erfindung beispielsweise der Lichtschutzfaktor (LSF bzw. SPF) dar.

Die kosmetischen und dermatologischen Zubereitungen im Sinne der vorliegenden Erfindung hinterlassen auf der Haut keinen schmierigen oder klebrigen Eindruck, sind ausgezeichnet hautverträglich und zeichnen sich ferner überraschender Weise dadurch aus, dass sie sandabweisend sind.

UV-Filtersubstanzen auf Basis von Triazinderivaten , welche das Strukturmotiv 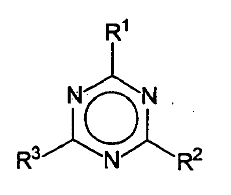 aufweisen, sind an sich bekannt und werden zum Beispiel in den der EP-A-775 698, der EP-A-0 878 469 und der EP-A-1 027 881 beschrieben.

Hinsichtlich der C₃-Achse des Triazingrundkörpers dieser Verbindungen sind sowohl symmetrische Substitution als auch unsymmetrische Substitution denkbar. In diesem Sinne symmetrisch substituierte s-Triazine weisen drei gleiche Substituenten R¹, R² und R³ auf, während unsymmetrisch substituierte s-Triazinderivate demzufolge unterschiedliche Substituenten aufweisen, wodurch die C₃-Symmetrie zerstört wird. Im Sinne der vorliegenden Erfindung wird als "unsymmetrisch" stets unsymmetrisch hinsichtlich der C₃-Achse des Triazingrundkörpers verstanden, es sei denn, etwas anderes wäre ausdrücklich erwähnt. Erfindungsgemäße unsymmetrisch substituierte s-Triazinderivate werden im Folgenden auch einfach als Triazinderivate bezeichnet.

Erfindungsgemäß ist in Komponente (a) 2,4-Bis-{[4-(2-ethyl-hexyloxy)-2-hydroxy]-phenyl}-6-(4-methoxyphenyl)-1 ,3,5-triazin (INCl: Aniso Triazin) enthalten, welches unter der Handelsbezeichnung Tinosorb® S bei der CIBA-Chemikalien GmbH erhältlich und durch folgende Struktur gekennzeichnet ist:

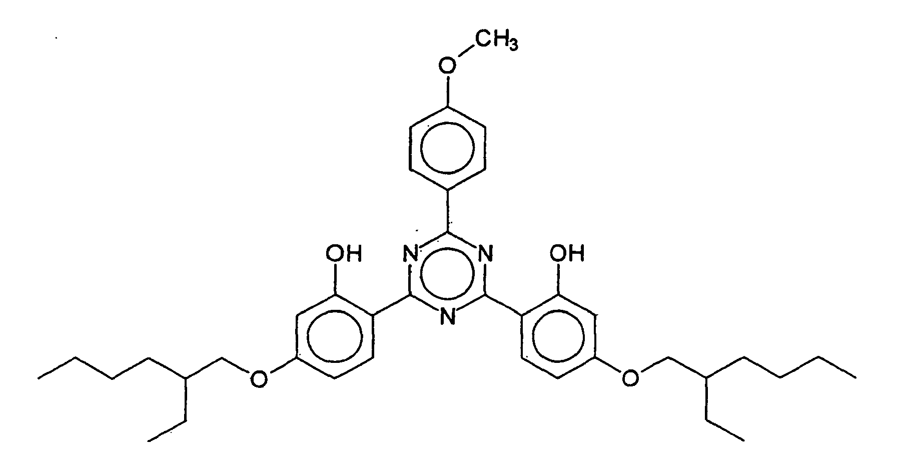

Das erfindungsgemäße unsymmetrisch substituierte s-Triazinderivat wird vorteilhaft in die Ölphase der kosmetischen oder dermatologischen Formulierungen eingearbeitet.

Weitere UV-A-Filtersubstanz der Komponente (a) der erfindungsgemäßen Zubereitungen ist das Phenylen-1,4-bis-(2-benzimidazyl)-3,3'-5,5'-tetrasulfonsäure-bis-natriumsalz 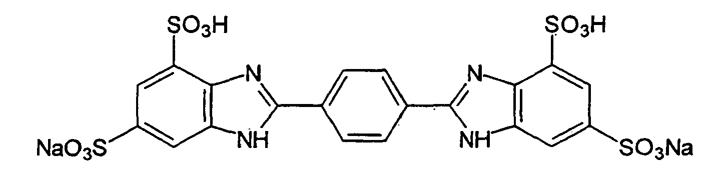 mit der INCl-Bezeichnung Bisimidazylate, welches beispielsweise unter der Handelsbezeichnung Neo Heliopan AP bei Haarmann & Reimer erhältlich ist.

Erfindungsgemäß werden in Komponente (a) also mindestens die beiden nachstehend genannten UV-Filtersubstanzen zusammen verwendet.

2,4-Bis-{[4-(2-ethyl-hexyloxy)-2-hydroxy]-phenyl}-6-(4-methoxyphenyl)-1,3,5-triazin mit Phenylen-1,4-bis-(2-benzimidazyl)-3,3'-5,5'-tetrasulfonsaure-bis-natriumsalz.

Eine weitere Ausführungsform der vorliegenden Erfindung enthält 2,4-Bis-{[4-(2-ethyl-hexyloxy)-2-hydroxy]-phenyl}-6-(4-methoxyphenyl)-1,3,5-triazin (Tinosorb® S), Phenylen-1,4-bis-(2-benzimidazyl)-3,3'-5,5'-tetrasulfonsäure-bis-natriumsalz (Neo Heliopan® AP) und 2,2'-Methylen-bis-(6-(2H-benzotriazol-2-yl)-4-(1,1,3,3-tetramethylbutyl)-phenol) (Tinosorb® M) zusammen als UV-Filtersubstanzen der Komponente (a).

Die oben genannten UV-Filtersubstanzen der Komponente (a) sind vorteilhaft insgesamt in Mengen von 0,1 Gew.-% bis 20 Gew.-%, vorzugsweise 0,5 bis 15 Gew.-%, insbesondere 1 bis 10 Gew.-% vorhanden, jeweils bezogen auf das Gesamtgewicht der Zubereitungen.

Die erfindungsgemäßen kosmetischen und dermatologischen Zubereitungen enthalten als zweite Komponente (b) der synergistischen Kombination mindestens einen Füllstoff, der aus synthetischen organischen Polymeren ausgewählt ist. Bei den Füllstoffen handelt es sich um partikuläre Stoffe, die keinen Farbeffekt im Produkt (d.h. der kosmetischen und dermatologischen Zubereitung selbst) oder auf der Haut hervorrufen.

Erfindungsgemäß geeignete synthetische organische Polymere, d. h. Polymerpartikel, welche in der Zubereitung in Form von Feststoffen vorliegen, sind beispielsweise Polycarbonate, Polyether, Polyethylen, Polypropylen, Polyvinylchlorid, Polystyrol, Polyamide, Polyurethane, Polyacrylate und dergleichen mehr. Besonders vorteilhaft ist z. B. die Substanz mit der INCI-Bezeichnung *HDI* / *Trimethylol Hexyllactone Crosspolymer,* welche unter der Bezeichnung BPD-500/Plastic Powder D von der Firma Kobo erhältlich ist.

Ferner vorteilhaft im Sinne der vorliegenden Erfindung ist Nylon (Polyamid 6 und Polyamid 12), wie beispielsweise mikrofeine Polyamid-Partikel, insbesondere die unter der Handelsbezeichnung SP-500 bei der Firma TORAY erhältlichen. Ferner vorteilhaft sind Polyamid 6- (auch: Nylon 6), bzw. Polyamid 12- (auch: Nylon 12), Partikel. Polyamid 6 ist das aus ε-Aminocapronsäure (6-Aminohexansäure), oder ε-Caprolactam aufgebaute Polyamid [Poly(ε-caprolactam)], und Polyamid 12 ist ein Poly(ε-laurinlactam), aus ε-Laurinlactam. Vorteilhaft im Sinne der vorliegenden Erfindung sind beispielsweise Orgasol^{®} 1002 (Polyamid 6), und Orgasol^{®} 2002 (Polyamid 12), von der Firma ELF ATOCHEM.

Weitere vorteilhafte organische Füllstoffe sind:
⇒ PMMA: Polymethylmethacrylate
⇒ Polyethylene Spheres
⇒ Polyurethane
⇒ Silikonharze: Trimethylsiloxysilicate (z. B. SR 1000 GE Bayer Silicones)
⇒ Silikonelastomere

So kann es z. B. von erheblichem Vorteil sein, solche Silikonelastomeren in Zubereitungen gemäß der vorliegenden Erfindung zu verwenden, wie sie beispielsweise in den US-Patenten US 4980167 oder US 4742142 beschrieben werden. Vorteilhafte Silikonelastomere sind ferner z. B. solche, welche unter den Namen KSG6 von Shin Etsu, Tefil E-505C oder Trefil E-506C von Dow Corning, Gransil von Grant Industries (SR-CYC, SR-DMF10, SR-DC556), vertrieben werden sowie solche, die in Form von vorgefertigten Gelen verkauft werden (wie z. B. KSG15, KSG17, KSG16, KSG18 von Shin Etsu, Gransil SP 5CYC Gel, Gransil SR DMF 10 Gel, Gransil SR DC 556 Gel, Gransil GCM, Gransil PM Gel, Gransil DMG-5, SF 1204 und JK 113 von Gereral Electric). Weitere vorteilhafte Silikonelastomere können gewählt werden aus der Gruppe der Vinyl Dimethicone Crosspolymere, wie z. B. das Dow Corning 9506 Cosmetic Powder von Dow Corning (INCI: Dimethicone / Vinyl Dimethicone Crosspolymer).

Weiterhin vorteilhaft eingesetzt werden können sogenannte Silikonharze, wie z.B. KSP-100, KSP-200 oder KSP-300 von Shin Etsu, die ebenfalls unter der INCI bezeichnung Dimethicone / Vinyl Dimethicone Crosspolymer geführt werden oder SR 1000 von GE Bayer Silicones, das die INCI Bezeichnung Trimethylsiloxy Silicate trägt

Zu den organischen Siliziumverbindungen gehören die Siloxan Elastomere und Siloxan Harze. Von denen sind besonders bevorzugt die KSP-Typen von Shin Etsu, sowie das Trimethylsiloxysilicate.

Weitere erfindungsgemäß bevorzugte Füllstoffe kommen aus der Gruppe der sphärischen Partikel. Besonders bevorzugt ist der mittlere Teilchendurchmesser kleiner als 20 µm. Weiterhin werden bevorzugt spärische Partikel organischen Ursprungs gewählt. Besonders bevorzugt ist davon das BPD-500, das von der Firma Kobo vertrieben wird.

Desweiteren sind bevorzugt sphärische Partikel mit einem mittlerern Teilchendurchmesser kleiner als 10 µm. Davon besonders bevorzugt sind Nylon-12, das z.B. als SP-501 oder SP-500 von der Firma Kobo vertrieben wird. Weiterhin bevorzugt sind Polymethylmethacrylate, die z.B. unter dem Handelsnamen Covabead LH 85 von LCW vertrieben wird.

Die erfindungsgemäßen Füllstoffe können sowohl einzeln als auch im Gemisch eingesetzt werden. Weiterhin ist die Kombination der erfindungsgemäßen organischen synthetischen Polymere mit in Wasser nicht quellbaren Schichtsilikaten oder sphärischen Siliziumdioxide vorteillhaft.

Die Gesamtmenge der Füllstoffe beträgt vorteilhaft 0,1 Gew.-% bis 30 Gew.-%, vorzugsweise 0,5 bis 15 Gew.-%, insbesondere 1,0 bis 10 Gew.-% gewählt, jeweils bezogen auf das Gesamtgewicht der Zubereitungen.

Weitere Füllstoffe, die neben denen der Komponente (b) in den erfindungsgemäßen Zubereitungen eingesetzt werden können, sind im Folgenden genannt:
Aerosile wie die unter den folgenden Handelsnamen erhältlichen Produkte Aerosil 130 (Degussa Hüls), Aerosil 200 (Degussa Hüls), Aerosil 255 (Degussa Hüls), Aerosil 300 (Degussa Hüls), Aerosil 380 (Degussa Hüls), B-6C (Suzuki Yushi), CAB-O-SIL Fumed Silica (Cabot), CAB-O-SIL EH-5 (Cabot), CAB-O-SIL HS-5 (Cabot), CAB-O-SIL LM-130 (Cabot), CAB-O-SIL MS-55 (Cabot), CAB-O-SIL M-5 (Cabot), E-6C (Suzuki Yushi), Fossil Flour MBK (MBK), MSS-500 (Kobo), Neosil CT 11 (Crosfield Co.), Ronasphere (Rona/EM Industries), Silica, Anhydrous 31 (Whittaker, Clark & Daniels), Silica, Crystalline 216 (Whittaker, Clark & Daniels), Silotrat-1 (Vevy), Sorbosil AC33 (Crosfield Co.), Sorbosil AC 35 (Crosfield Co.), Sorbosil AC 37 (Crosfield Co.), Sorbosil AC 39 (Crosfield Co.), Sorbosil AC77 (Crosfield Co.), Sorbosil TC 15 (Crosfield Co.), Spherica (Ikeda), Spheriglass (Potters-Ballotini), Spheron L-1500 (Presperse), Spheron N-2000 (Presperse), Spheron P-1500 (Presperse), Wacker HDK H 30 (Wacker-Chemie), Wacker HDK N 20 (Wacker-Chemie), Wacker HDK P 100 H (Wacker Silicones), Wacker HDK N 20P (Wacker-Chemie), Wacker HDK N 25P (Wacker-Chemie), Wacker HDK S 13 (Wacker-Chemie), Wacker HDK T 30 (Wacker-Chemie), Wacker HDK V 15 (Wacker-Chemie), Wacker HDK V 15 P (Wacker-Chemie), Zelec Sil (DuPont).

Ferner sind Bornitride geeignet, insbesondere die folgenden Handelsprodukte:

| **Handelsname** | **erhältlich bei:** |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN^{®} | Carborundum |
| Wacker-Bornitrid BNP | Wacker-Chemie |

Weitere zusätzlich in die erfindungsgemäßen Zubereitungen einarbeitbare Füllstoffe sind Carbonate, wie z. B. Magnesiumcarbonat (MgCO₃) und Calciumcarbonat (CaCO₃). Carbonate werden erfindungsgemäß insbesondere als Füllstoffe in trockenen Pudern verwendet.

Natürliche organische Polymere wie Seidenpuder, mikrokristalline Cellulose und/oder Zinkstearate können ebenfalls zusätzlich verwendet werden.

Vorteilhafte organische Füllstoffe sind ferner Stärke und Stärkederivate, wie:
⇒ Maisstärke Zea Mays (Amidon De Mais MST (Wackherr), Argo Brand Corn Starch (Corn Products), Pure-Dent (Grain Processing), Purity 21C (National Starch)),
⇒ Reisstärke (D.S.A. 7 (Agrana Stärke), Oryzapearl (Ichimaru Pharcos)),
⇒ Distarch Phosphate (Corn PO4 (Agrana Stärke), Corn PO4 (Tri-K)),
⇒ Sodium Corn Starch Octenylsuccinate (C* EmCap - Instant 12639 (Cerestar USA)),
⇒ Aluminium Starch Octenylsuccinate (Covafluid AMD (Wackherr), Dry Flo-PC (National Starch), Dry Flo Pure (National Starch), Fluidamid DF 12 (Roquette)).

Diese zusätzlichen Füllstoffe können insgesamt in solchen Mengen vorliegen, wie oben für die erfindungsgemäßen Füllstoffe der Komponente (b) angegeben.

Weitere herkömmliche UV-Filtersubstanzen, die neben denen der Komponente (a) in den erfindungsgemäßen Zubereitungen eingesetzt werden, sind im Folgenden genannt:
Vorteilhafte öllösliche UV-B-Filter sind z. B.:
   - 3-Benzylidencampher-Derivate, vorzugsweise 3-(4-Methylbenzyliden)campher [INCl: 4-Methylbenzylidene Camphor], welches von Merck unter der Warenbezeichnung Eusolex 6300 vertrieben wird und/oder 3-Benzylidencampher;
   - 4-Aminobenzoesäure-Derivate, vorzugsweise 4-(Dimethylamino)-benzoäsäure(2-ethylhexyl)ester, 4-(Dimethylamino)benzoesäureamylester;
   - Ester der Zimtsäure, vorzugsweise 4-Methoxyzimtsäure(2-ethylhexyl)ester, 4-Methoxyzimtsäureisopentylester;
   - Ester der Salicylsäure, vorzugsweise Salicylsäure(2-ethylhexyl)ester, Salicylsäure(4-isopropylbenzyl)ester, Salicylsäurehomomenthylester,
   - Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
   - Ester der Benzalmalonsäure, vorzugsweise 4-Methoxybenzalmalonsäuredi(2-ethylhexyl)ester,
   - hinsichtlich der C₃-Achse des Triazingrundkörpers symmetrisch Triazinderivate, vorzugsweise 4,4',4"-(1,3,5-Triazin-2,4,6-triyltriimino)-tris-benzoesäure-tris(2-ethylhexylester) [INCl: Octyl Triazone], welches von der BASF Aktiengesellschaft unter der Warenbezeichnung UVINUL® T 150 vertrieben wird,
   - Benzotriazolderivate, vorzugsweise 2,2'-Methylen-bis-(6-(2H-benzotriazol-2-yl)-4-(1,1,3,3-tetramethylbutyl)-phenol)
   - sowie an Polymere gebundene UV-Filter.

Vorteilhafte wasserlösliche UV-B-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Weitere herkömmliche, in erfindungsgemäßen Zubereitungen verwendbare UV-A-Filter sind beispielsweise Derivate des Dibenzoylmethans, insbesondere 1-(4'-tert.Butylphenyl)-3-(4'-methoxyphenyl)propan-1,3-dion und um 1-Phenyl-3-(4'-isopropylphenyl)propan-1,3-dion. Vorteilhafte UV-A-Filtersubstanz ist ferner das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Ferner vorteilhaft sind das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

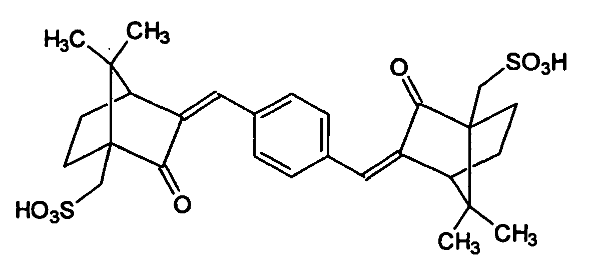

Vorteilhafte Filtersubstanzen, die sowohl UV-A- als auch UV-B-Strahlung absorbieren, sogenannte Breitbandfilter, sind z.B. 2,2'-Methylen-bis-(6-(2H-benzotriazol-2-yl)-4-(1,1,3,3-tetramethylbutyl)-phenol) oder 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 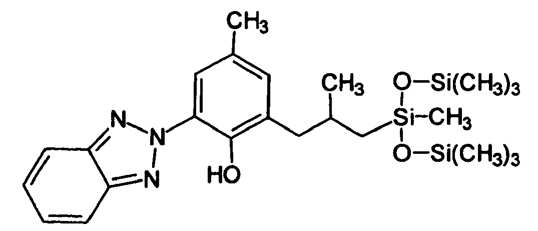 gekennzeichnet ist.

Ferner sind beispielsweise bestimmte Salicylsäurederivate wie 4-lsopropylbenzylsalicylat, 2-Ethylhexylsalicylat (= Octylsalicylat), Homomenthylsalicylat als UV-Filtersubstanzen geeignet.

Eine weitere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist und sich durch folgende Struktur auszeichnet:

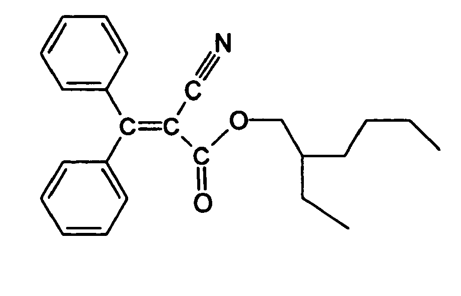

Ferner können die erfindungsgemäßen Zubereitungen, wenn sie in Form von sogenannten ölfreien kosmetischen oder dermatologischen Zubereitungen vorliegen, welche eine Wasserphase und mindestens eine bei Raumtemperatur flüssige UV-Filtersubstanz als weitere Phase enthalten, die folgenden bei Raumtemperatur flüssigen UV-Filtersubstanzen enthalten.

Vorteilhafte bei Raumtemperatur flüssige UV-Filtersubstanzen sind Homomenthylsalicylat, 2-Ethylhexyl-2-cyano-3,3-diphenylacrylat, 2-Ethylhexyl-2-hydroxybenzoat und Ester der Zimtsäure, vorzugsweise 4-Methoxyzimtsäure(2-ethylhexyl)ester und 4-Methoxyzimtsäureisopentylester.

Homomenthylsalicylat (INCl: Homosalate) zeichnet sich durch die folgende Struktur aus:

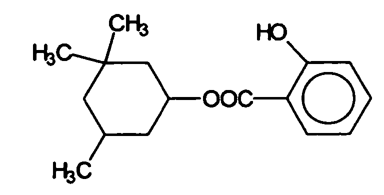

2-Ethylhexyl-2-cyano-3,3-diphenylacrylat (INCI: Octocrylene) ist von BASF unter der Bezeichnung Uvinul^{®} N 539 erhältlich und zeichnet sich durch folgende Struktur aus:

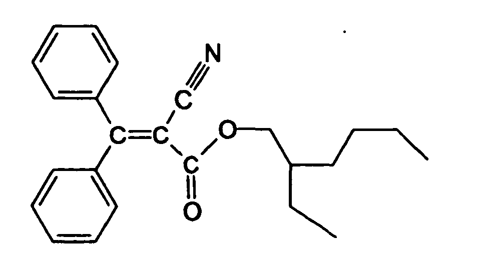

2-Ethylhexyl-2-hydroxybenzoat (2-Ethylhexylsalicylat, Octylsalicylat, INCl: Octyl Salicylate) ist beispielsweise bei Haarmann & Reimer unter der Handelsbezeichnung Neo Heliopan OS erhältlich und zeichnet sich durch die folgende Struktur aus:

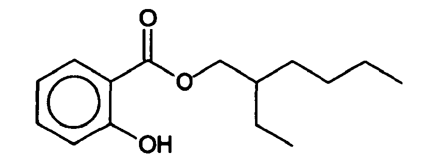

4-Methoxyzimtsäure(2-ethylhexyl)ester (2-Ethylhexyl-4-methoxycinnamat, INCl: Octyl Methoxycinnamate) ist beispielsweise bei Hoffmann-La Roche unter der Handelsbezeichnung Parsol MCX erhältlich und zeichnet sich durch die folgende Struktur aus:

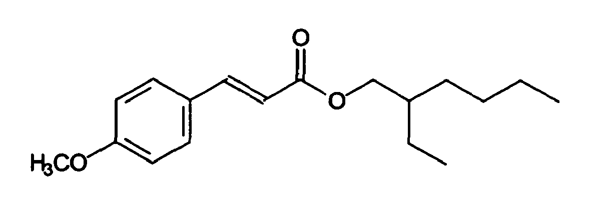

4-Methoxyzimtsäureisopentylester (Isopentyl-4-methoxycinnamat, INCl: Isoamyl p-Methoxycinnamate) ist beispielsweise bei Haarmann & Reimer unter der Handelsbezeichnung Neo Heliopan E 1000 erhältlich und zeichnet sich durch die folgende Struktur aus:

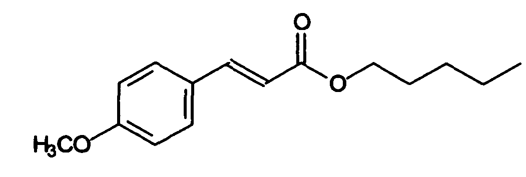

Die Gesamtmenge an einer oder mehreren bei Raumtemperatur flüssigen UV-Filtersubstanzen in den fertigen kosmetischen oder dermatologischen Zubereitungen wird vorteilhaft aus dem Bereich 0,1 Gew.-% bis 30 Gew.-%, vorzugsweise von 0,5 bis 20 Gew.-% gewählt, jeweils bezogen auf das Gesamtgewicht der Zubereitungen.

Die Liste der genannten herkömmlichen UV-Filtersubstanzen, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen.

Die kosmetischen und dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Konservierungsmittel, Konservierungshelfer, Bakterizide, Parfüme, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel sind beispielsweise Formaldehydabspalter (wie z. B. DMDM Hydantoin), lodopropylbutylcarbamate (z. B. die unter den Handelsbezeichnungen Koncyl-L, Koncyl-S und Konkaben LMB von der Fa. Lonza erhältlichen), Parabene, Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Zitronensäure, Milchsäure, Apfelsäure), Huminsäure, Gallensäure, Gallenextrakte, Bilirubin, Biliverdin, EDTA, EGTA, IDS (Iminodibernsteinsäure) und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Eine erstaunliche Eigenschaft der erfindungsgemäße Zubereitungen ist, dass diese sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Zubereitung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Zubereitung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z. B. Hydrocortison-17-valerat, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die gamma-Linolensäure, Ölsäure, Eicosapentaensäure, Docosahexaënsäure und deren Derivate, Chloramphenicol, Coffein, Prostaglandine, Thymol, Campher, Extrakte oder andere Produkte pflanzlicher und tierischer Herkunft, z. B. Nachtkerzenöl, Borretschöl oder Johannisbeerkernöl, Fischöle, Lebertran aber auch Ceramide und ceramidähnliche Verbindungen und so weiter.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspositionen angegeben):

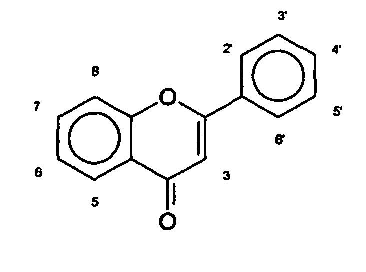

Einige der wichtigeren Flavone, welche auch bevorzugt in erfindungsgemäßen Zubereitungen eingesetzt werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt aus der Gruppe der Substanzen der generischen Strukturformel 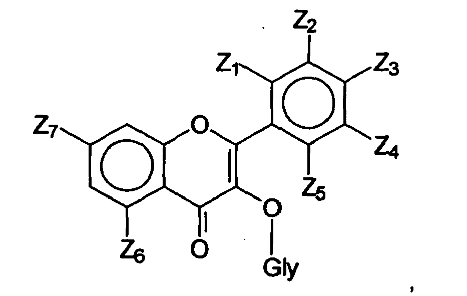 wobei Z₁ bis Z₇ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 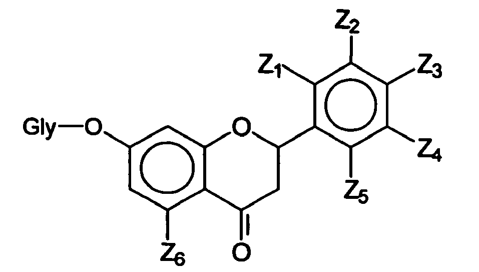 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 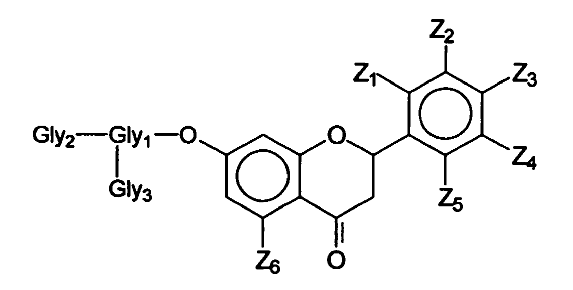 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur

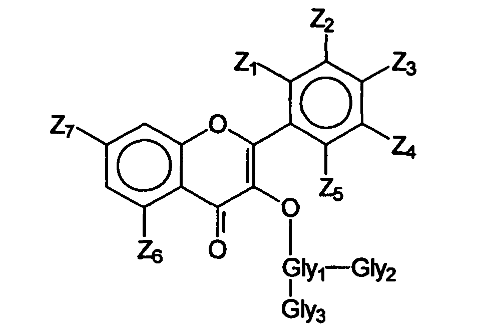

Besonders vorteilhaft werden die erfindungsgemäßen Flavonglycoside aus der Gruppe, welche durch die folgende Struktur wiedergegeben werden: 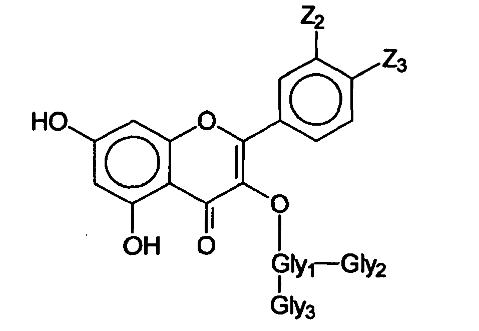 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-(3-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomarein (3',4',7,8Tetrahydroxyflavanon-7-glucosid) und Isoquercetin (3,3',4',5,7-Pentahydroxyflavanon-3-(β-D-Glucopyranosid).

Vorteilhaft ist es auch, dem oder die Wirkstoffe aus der Gruppe der Ubichinone und Plastochinone zu wählen.

Ubichinone zeichnen sich durch die Strukturformel 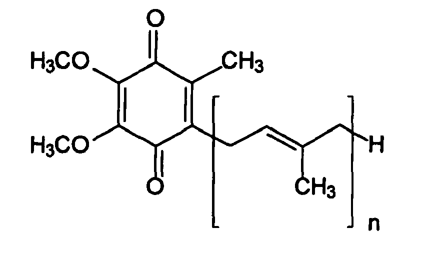 aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

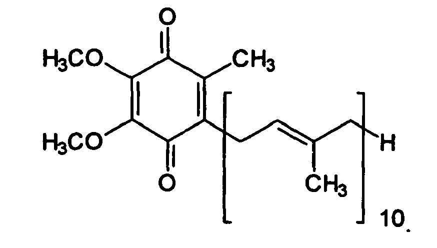

Plastochinone weisen die allgemeine Strukturformel 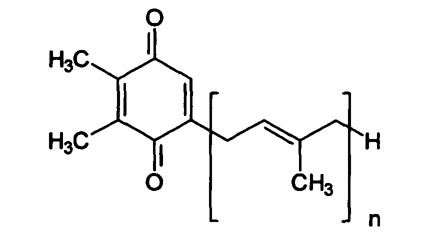 auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung. Kreatin zeichnet sich durch folgende Struktur aus:

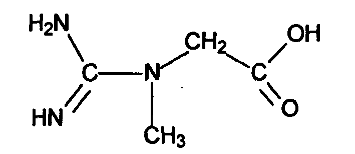

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist L-Carnitin [3-Hydroxy-4-(trimethylammonio)-buttersäurebetain]. Auch Acyl-Carnitine, welche gewählt aus der Gruppe der Substanzen der folgenden allgemeinen Strukturformel 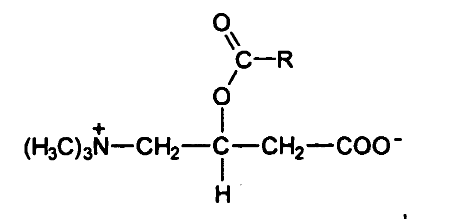 wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Wirkstoffe sind Sericosid, Pyridoxol, Vitamin K, Biotin und Aromastoffe.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Die erfindungsgemäßen Zubereitungen können in Form von Wasser-in-Öl-(W/O) oder Öl-in-Wasser-(O/W)-Emulsionen, komplexen Emulsionen (O/W/O, W/O/W), als Öl-in-Alkohol-Lotion, als vesikulare Dispersion eines ionischen oder nichtionischen amphiphilen Lipids, als Gel, feste wasserfreie oder wasserhaltige Stifte oder als loses oder gepresstes Puder vorliegen.

Vorzugsweise sind erfindungsgemäße Zubereitungen Emulsionen oder wasserfreie Zubereitungen, insbesondere Stifte.

Die Ölphase der erfindungsgemäßen Zubereitungen wird vorteilhaft ausgewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkemöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether und/oder Dicaprylylcarbonat, beispielsweise das unter der Handelsbezeichnung Cetiol CC bei der Fa. Cognis erhältliche.

Es ist ferner bevorzugt, das oder die Ölkomponenten aus der Gruppe Isoeikosan, Neopentylgtykoldiheptanoat, Propylenglykoldicaprylatldicaprat, Caprylic/Capric/Diglycerylsuccinat, Butylenglykol Caprylat/Caprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon *(Hallstar AB)* und/oder Diethylhexylnaphthalat (Hallbrite/*Corapan TQ*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 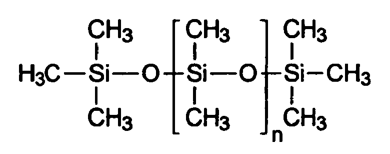 werden auch als Polydimethylsiloxan bzw. Dimethicon (INCl) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCl auch als Cyclomethicone bezeichnet werden, aminomodifizierte Silikone (INCl: Amodimethicone) und Silikonwachse, z. B. Polysiloxan-Polyalkylen-Copolymere (INCl: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Erfindungsgemäß vorteilhaft sind ferner z. B. natürliche Wachse tierischen und pflanzlichen Ursprungs, wie beispielsweise Bienenwachs, Chinawachs, Hummelwachs und andere Insektenwachse sowie Sheabutter.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Auch Moisturizer können bevorzugt verwendet werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Farbstoffe und/oder Farbpigmente enthalten, insbesondere wenn sie in Form von dekorativen Kosmetika vorliegen.

Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 20 Gew.-%, insbesondere von 1,0 bis 15 Gew.-% gewählt, jeweils bezogen auf das Gesamtgewicht der Zubereitungen.

Die Pigmente können auch vorteilhaft als UV-Filtersubstanzen wirken.

Ferner kann es gegebenenfalls von Vorteil sein, Filmbildner in die erfindungsgemäßen kosmetischen oder dermatologischen Zubereitungen einzuarbeiten, beispielsweise um die Wasserfestigkeit der Zubereitungen zu verbessern oder die UV-Schutzleistung zu erhöhen (UV-A- und/oder UV-B-Boosting). Geeignet sind sowohl wasserlösliche bzw. dispergierbare als auch fettlösliche Filmbildner, jeweils einzeln oder in Kombination miteinander.

Vorteilhafte wasserlöslich bzw. dispergierbare Filmbildner sind z. B. Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF), Acrylat-Copolymere (Dermacryl 79, National Starch) etc.

Vorteilhafte fettlösliche Filmbildner sind z. B., die Filmbildner aus der Gruppe der Polymere auf Basis von Polyvinylpyrrolidon (PVP)

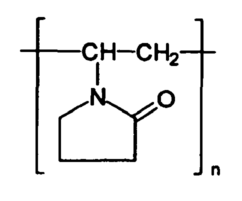

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Die erfindungsgemäßen kosmetischen oder dermatologischen Lichtschutzformulierungen können dem kosmetischen oder dermatologischen Lichtschutz, z.B. als Sonnenschutzmittel, ferner zur Behandlung, Pflege und Reinigung der Haut und/oder der Haare und insbesondere als Schminkprodukt in der dekorativen Kosmetik dienen.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Erfindungsgemäß sind ferner kosmetische und dermatologische Zubereitungen, deren hauptsächlicher Zweck nicht der Schutz vor Sonnenlicht ist, die aber dennoch einen Gehalt an UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Darüber hinaus eignen sich ausgewählte erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, insbesondere zur Prophylaxe und Behandlung kosmetischer oder dermatologischer Hautveränderungen, wie sie z. B. bei der Hautalterung auftreten. Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff Senile Xerosis" fallen können:
a) Trockenheit, Rauhigkeit und Ausbildung von (Trockenheits-) Fältchen,
b) Juckreiz und
c) verminderte Rückfettung durch Talgdrüsen (z. B. nach dem Waschen).
   Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

In einer besonderen Ausführungsform betrifft die vorliegende Erfindung daher insbesondere Produkte zur Pflege der auf natürliche Weise gealterten Haut, sowie zur Behandlung der Folgeschäden der Lichtalterung, insbesondere der unter a) bis g) aufgeführten Phänomene.

Zur Anwendung werden die kosmetischen und dermatologischen Zubereitungen in der für Kosmetika üblichen Weise auf die Haut und/oder die Haare in ausreichender Menge aufgebracht.

Die Herstellung der erfindungsgemäßen Zubereitungen erfolgt nach im Stand der Technik bekannten Methoden, die z.B. Uhlamann's Enzyclopedia of Industrial Chemistry, Vol A 24 Skin Cosmetics, VCH Verlagsgesellschaft, Weinheim (1993) auf den Seiten 219 ff beschrieben sind und die darin zitierte Literatur.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### Beispielrezeptur wasserhaltige Stifte

| **INCl** | **Lippenpflegestift** |
|---|---|
| Rizinusöl | ad.100 |
| Capryl-/Caprinsäure Triglycerid | 20,0 |
| Octyldodecanol | 20,0 |
| Paraffinöl | |
| Pentaerythrityl Tetraisostearat | |
| Carylylcarbonat | |
| Isopopylpalmitat | |
| Jojobaöl | 6,0 |
| Lanolin Öl | 5,0 |
| Simethicon | |
| PEG-45/ Dodecyl Glycol Copolymer | |
| Polyglyceryl-3 Diisostearat | 3,7 |
| Bis-Diglyceryl Polyacyladipat-2 | |
| Cetearyl Alkohol | 6,0 |
| Cetylpalmitat | 7,5 |
| C16-36 Alkyl Stearat | |
| C18-38 Alkyl Hydroxystearoyl Stearat | |
| C20-40 Alkyl Stearat | 16,0 |
| Carnaubawachs | |
| Bienenwachs | 6,0 |
| PVP / Eicosen Copolymer | |
| mikronisiertes Titandioxid | |
| Ethylhexyl Methoxycinnamat | |
| Bis-Ethylhexyloxyphenol methoxyphenyl Triazin | 2,00 |
| Butyl Methoxydibenzoylmethan | |
| Phenyl, Dibenzimidazol Tetrasulfonsäure | 0,50 |
| Ethylhexyl Triazon | |
| Octocrylen | 7,50 |
| Diethylhexyl Butamido Triazon | |
| Phenylbenzmidazol Sulfonsäure | 2,00 |
| Methylen Bis-Benzotriazolyl Tetramethylbutylphenol | 0,50 |
| Ethylhexyl Salicylat | |
| Homosalat | |
| Diethylhexyl-2,6-naphthalat | |
| Zinkoxid NDM | 2,00 |
| Zinkoxid Z-Cote HP-1 | |
| Nylon-12 | 3,0 |
| BiOCl₃ | |
| Vinyl Dimethicone Crosspolymere | |
| Lauroyl Lysin | 0,5 |
| Polymethylsilsesquioxan | |
| Poröses PMMA (Covabead LH 85) | |
| PMMA | |
| Interferenz Pigmente | |
| Titandioxid, Cl 77891 | 1,0 |
| Eisenoxide Cl 77491, 77492, 77499 | |
| D&C Rot 7 | |
| D&C Rot 6 | |
| FDBC Blau 1 | |
| D&C Rot 21 | |
| FD&C Gelb 6 | |
| D&C Rot 28 | 0,05 |
| D&C Rot 34 | |
| Diammonium Citrat | 0,08 |
| Zitronensäure | 0,05 |
| Glycerin | |
| Panthenol | |
| Parfum, Konservierungsmittel, BHT, Neutralisationsmittel, Sequestriemittel | q.s |
| Wasser | 2,5 |

### Beispielrezeptur Lippenstifte

| | **1** | **2** |
|---|---|---|
| Mikrokristallines Wachs | 3,5 | 3,0 |
| Ozokerit | | |
| Carnaubawachs | 3,2 | 1,5 |
| Candelillawachs | 8,0 | 4,2 |
| Bienenwachs | 0,6 | |
| C24-40 Alkyl Stearat | | 12,0 |
| Cetyl Ricinoleat | | |
| Cetyl Alkohol | 1,5 | |
| Lanolinöl | 10,0 | |
| Bis-Diglyceryl Polyacyladipat-2 | | 16,0 |
| Caprylic/Capric Triglycerid | 4,0 | |
| Avocadoöl | | |
| Isopropyl Palmitate | 4,0 | |
| Isostearyl Isostearat | | |
| Triisostearin | | 3,0 |
| Oleyl Erucat | | |
| C12-C15 Alkyl Benzoate | | |
| Pentaerythrityl Tetraisostearat | | 3,0 |
| Myristyl Lactat | | 1,0 |
| Jojobaöl | | |
| Octyldodecanol | 6,0 | 2,0 |
| Polyisobuten | | |
| hydriertes Polydecen | 4,0 | 3,0 |
| Squalan | 2,0 | |
| PVP/Hexadecen Copolymer | 3,0 | |
| PVP/Eicosen Copolymer | | |
| Lecithin | | |
| Polyglyceryl-2 Dipolyhydroxystearat | | |
| Polyglyceryl-3 Diisostearate | | 0,5 |
| Mikronisiertes Titandioxid | 2,0 | 0,5 |
| Ethylhexyl Methoxycinnamat | 4,50 | |
| Bis-Ethylhexyloxyphenol methoxyphenyl Triazin | 2,50 | 1,00 |
| Butyl Methoxydibenzoylmethan | | |
| Phenyl Dibenzimidazol Tetrasulfonsäure | 3,00 | 2,00 |
| Ethylhexyl Triazon | | |
| Octocrylen | 3,00 | 2,00 |
| Diethylhexyl Butamido Triazon | 1,50 | |
| Phenylbenzmidazol Sulfonsäure | | |
| Methylen Bis-Benzotriazolyl Tetramethylbutylphenol | | |
| Diethylhexyl-2,6-naphthalat | | |
| Disteardimonium Hectorite | | |
| Propylen Carbonat | | |
| Silica Dimethyl Silylate (Aerosil R972) | | |
| PMMA | | 5,0 |
| Bornitrid | | |
| Nylon-12 | 1,0 | |
| Vinyl Dimethicone Crosspolymere | | |
| Polymethylsilsesquioxan | 5,0 | |
| Interferenz Pigmente | 0,6 | |
| Titandioxid Cl 77891 | | 2,0 |
| Eisenoxide Cl 77491, 77492, 77499 | 1,6 | 3,5 |
| D&C Rot 7 | 0,6 | 3,5 |
| D&C Rot 6 | | |
| FD&C Blau 1 | | |
| D&C Rot 33 | | 0,75 |
| D&C Rot 21 | | |
| FD&C Gelb 6 | 0,2 | |
| D&C Rot 30 | | 0,75 |
| D&C Rot 28 | | 0,5 |
| D&C Rot 34 | | |
| Tocopheryl Acetate | 1 | 1,5 |
| Ubichinon | | |
| Parfüm, Konservierungsmittel, Antioxidantien, etc. | q.s. | q.s. |
| Rizinusöl | ad 100 | ad 100 |

### Beispielrezeptur O/W Emulsionen

| **Inhaltsstoffe** | **Maskara** |
|---|---|
| PEG-30 Stearat | |
| PEG-40 Stearat | |
| PEG-100 Stearat | |
| Gylceryl Stearat | |
| Ceteareth-20 | |
| Polyglyceryl-3 Methylglucose Stearat | 3,5 |
| Sorbitan Stearat | 1,5 |
| Stearinsäure | |
| Glyceryl Stearat Citrat | |
| Cetyl Alkohol | 1,0 |
| Lecithin | |
| Getylpalmitat | |
| Veegum K = Mg-Al-Silikat | |
| Xanthan Gummi | |
| Carbomer | |
| Hydroxyethylcellulose | 0,1 |
| Capryl- / Caprinsäure Triglycerid | 3,0 |
| Dicaprylylether | 3,0 |
| Octyldodecanol | 3,0 |
| Dimethicon | 3,0 |
| Cyclomethicon | 3,0 |
| C12-C15 Alkyl Benzoat | |
| Cetearyloctanoat | |
| Squalan | |
| Isopropylpalmitat | |
| PPG -15 Stearylether | |
| Vaseline | |
| Hydrierte Kokosglyceride | |
| Hydriertes Polydecen | |
| Stearyl Dimethicon | |
| Ethylhexyl Methoxycinnamat | |
| Bis-Ethylhexyloxyphenol methoxyphenyl Triazin | 4,00 |
| Butyl Methoxydibenzoylmethan | |
| Phenyl Dibenzimidazol Tetrasulfonsäure | 1,00 |
| Ethylhexyl Triazon | 4,00 |
| Octocrylen | |
| Diethylhexyl Butamido Triazon | 1,00 |
| Phenylbenzmidazol Sulfonsäure | 1,00 |
| Methylen Bis-Benzotriazolyl Tetramethylbutylphenol | |
| Ethylhexyl Salicylat | |
| Diethylhexyl-2,6-naphthalat | |
| Distärke Phosphat | |
| Silizium Dioxid | |
| PMMA | |
| Nylon-12 | 5,0 |
| Lauroyl Lysin | 0,5 |
| Kaolin | 1,0 |
| Dimethicon / Vinyl Dimethicon Crosspolymer | |
| Polymethylsilsesquioxan | |
| Eisenoxide Cl 77491, 77492, 77499 | 8,0 |
| Titandioxid, Cl 77891 | |
| Ultramarin, Cl 77007 | 2,0 |
| Chromoxid, Cl 77288 | 0,6 |
| Interferenz Pigmente | |
| Glycerin | 5,0 |
| Parfum, Antiöxidans, Neutralisationsmittel, Sequestriermittel, Konservierungsmittel | q.s. |
| Wasser | ad 100 |

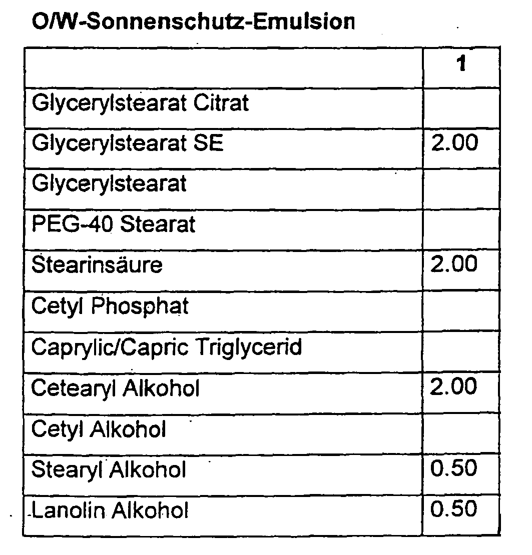

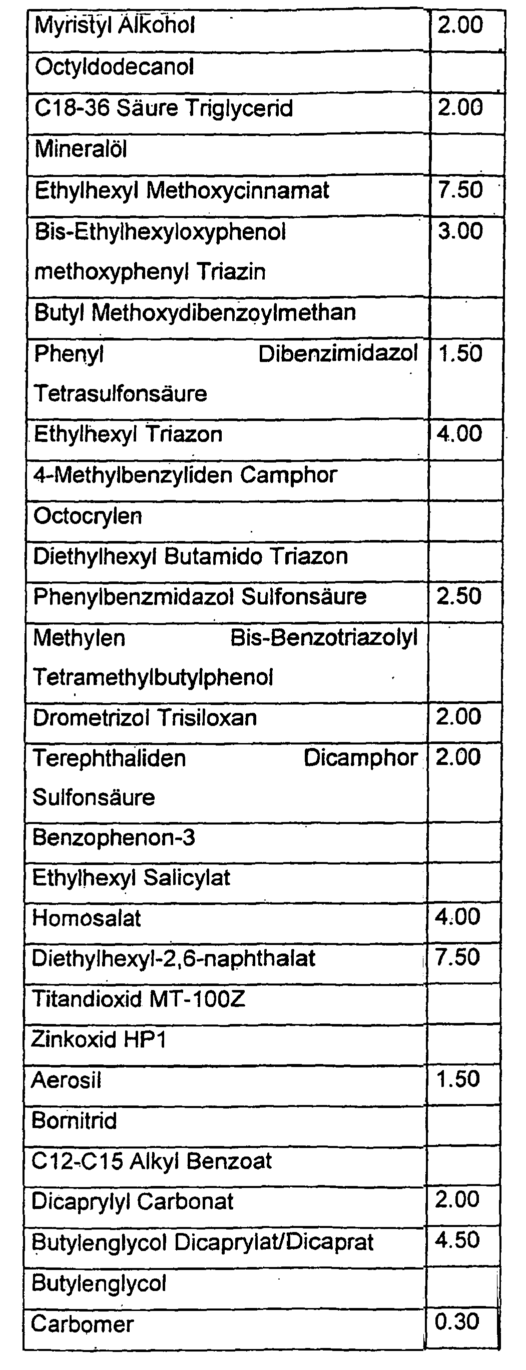

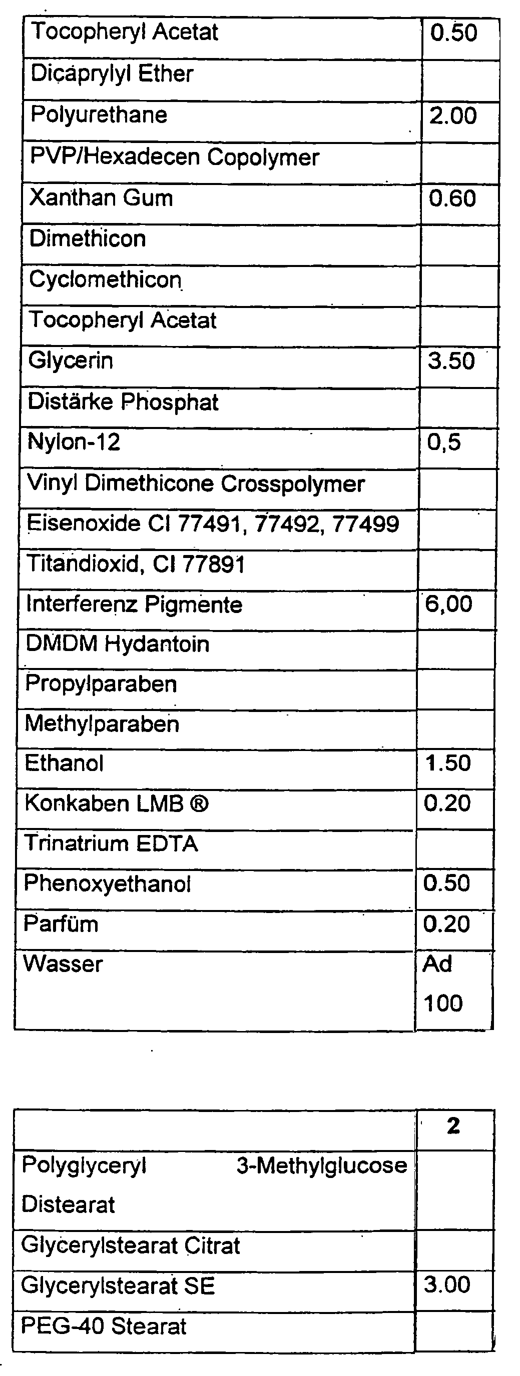

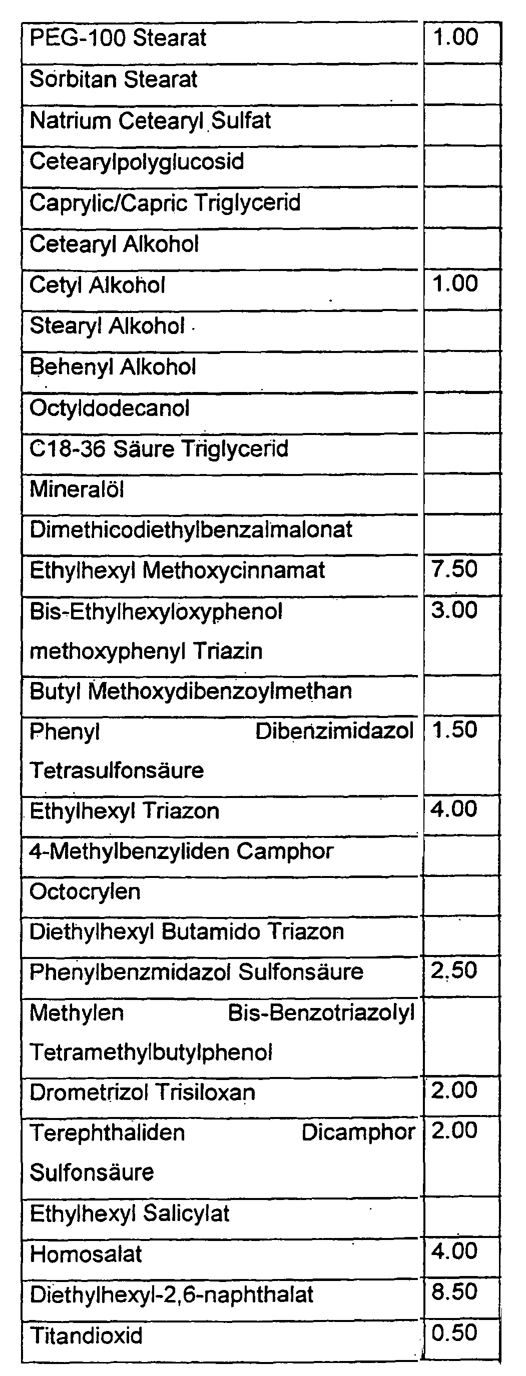

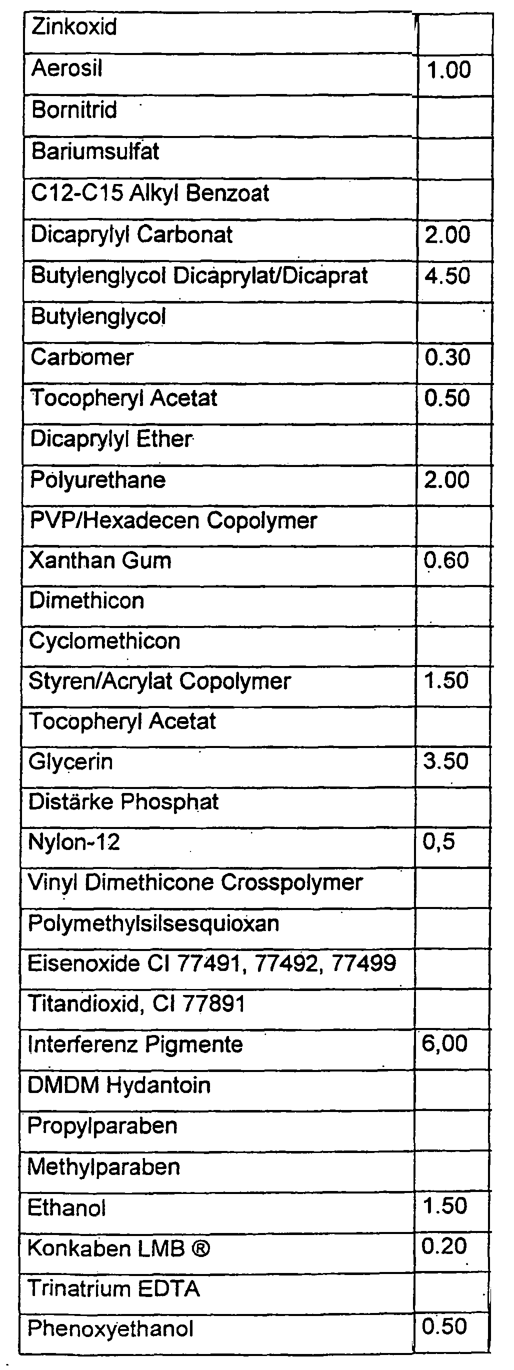

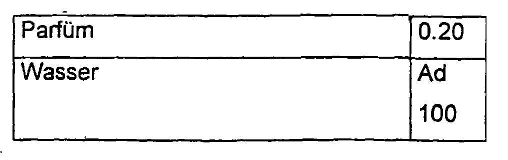

### W/O-Emulsionen

| | **1** | **2** |
|---|---|---|
| Triglycerinisostearat | 1,0 | |
| Diglycerindipolyhydroxystearat | 3,0 | |
| PEG-30 Dipolyhydroxystearat | | 5,0 |
| Cetyldimethiconcopolyol | | 1,0 |
| Paraffinöl | 12,5 | 17,5 |
| Vaseline | 8,0 | 2,5 |
| hydrierte Kokosglyceride | 2,0 | 0,25 |
| Decyloleat | 0,5 | 0,25 |
| Octyldodecanol | 0,5 | 0,25 |
| Aluminiumstearat | 0,4 | 0,05 |
| Magnesiumstearat | 0,1 | 1,0 |
| hydriertes Rizinusöl | 0,5 | 5,0 |
| Cyclomethicon | 1,0 | 1,0 |
| Phenyltrimethicon | | |
| hydriertes Polyisobuten | 0,5 | 3,0 |
| Capryl/Caprinsäuretriglycerid | 3,0 | 10,0 |
| Magnesiumsulfat | 0,5 | 1,0 |
| Glycerin | 3,0 | 1,5 |
| Ethylhexyl Methoxycinnamat | | |
| Bis-Ethylhexylöxyphenol methoxyphenyl Triazin | 1,50 | 3,00 |
| Butyl Methoxydibenzoylmethan | | 2,00 |
| Phenyl Dibenzimidazol Tetrasulfonsäure | 0,50 | 4,00 |
| Ethylhexyl Triazon | | 1,50 |
| Octocrylen | 4,00 | |
| Diethylhexyl Butamido Triazon | | |
| Phenylbenzmidazol Sulfonsäure | | |
| Methylen Bis-Benzotriazolyl Tetramethylbutylphenol | | |
| Diethylhexyl-2,6-naphthalat | | 4,50 |
| Zinkoxid NDM | 4,50 | |
| Titandioxid, Cl 777891 | 3,6 | 5,2 |
| Eisenoxide, Cl 77491, 77492, 77499 | 2,2 | 2,8 |
| Ultramarin; Cl 77007 | 0,4 | 0,4 |
| Quatemium-18 Hectorit | | |
| Propylen Carbonat | | |
| Natrium Stärke Octenylsuccinat | 0,25 | 0,75 |
| Nylon-12 | 2,0 | |
| Vinyl Dimethicone Crosspolymer | | 2,5 |
| Bornitrid | | |
| Parfum, Konservierungsmittel, Zitronensäure | q.s. | q.s. |
| Wasser | ad 100 | ad 100 |

### Beispielrezeptur Maskara

| | **1** |
|---|---|
| Isoparaffin C₁₀₋₁₂ | |
| Stearinsäure | 3,5 |
| Paraffinwachs | 4,0 |
| Carnaubawachs | 3,0 |
| Bienenwachs | 3,0 |
| Neutralöl | 3,0 |
| Cyclomethicon | |
| Lanolin Alkohol | |
| Distärke Phosphat | 2,00 |
| Nylon-12 | |
| Vinyl Dimethicone Crosspolymer | |
| Farbpigmente (Eisenoxide) | 8,0 |
| Ethylhexyl Methoxycinnamat | |
| Bis-Ethylhexyloxyphenöl methoxyphenyl Triazin | 1,25 |
| Butyl, Methoxydibenzoylmethan | |
| Phenyl Dibenzimidazol Tetrasulfonsäure | 0,50 |
| Ethylhexyl Triazon | 0,5 |
| Octocrylen | |
| Phenylbenzmidazol Sulfonsäure | |
| Methylen Bis-Benzotriazolyl Tetramethylbutylphenol | |
| Dimethicodiethylbenzalmalonat | 5,00 |
| Diethylhexyl-2,6-naphthalat | |
| Perlglanzpigmente | |
| Stearoxydimethicone (Belsil SM 6021 VP 621, Wacker) | |
| Polyalkoxy (C18-C36) dimethicone (176-1,0877, General Electric) | |
| PVP Copolymer | 3,0 |
| PVP/ VA Copolymer | |
| Quaternium-18-Hectorite | |
| Butandiol | 2,0 |
| Carbomer | |
| Hydroxyethylcellulose | |
| Ethanol | |
| Panthenol | 1,3 |
| Parfüm, Konservierungsmittel, Neutralisationsmittel, Antioxidantien, Sequestriermittel, etc. | q.s. |
| Wasser, demineralisiert | ad 100 |

## Patentansprüche

1. Kosmetische Zubereitung, **dadurch gekennzeichnet, dass** sie
(a) mindestens 2 UV-Filtersubstanzen enthält, wobei die erste UV-Filtersubstanz2,4-Bis-{[4-(2-ethyl-hexyloxy)-2-hydroxy]-phenyl}-6-(4-methoxyphenyl)-1,3,5-triazin und die zweite UV-Filtersubstanz in Komponente (a) Phenylen-1,4-bis-(2-benzimidazyl)-3,3'-5,5'-tetrasulfonsäure-bis-natriumsalz ist, und
(b) mindestens einen Füllstoff, ausgewählt aus der Gruppe von synthetischen organischen Polymeren
enthält.

2. Dermatologische Zubereitung, **dadurch gekennzeichnet, dass** sie
(a) mindestens 2 UV-Filtersubstanzen enthält, wobei die erste UV-Filtersubstanz 2,4-Bis-{[4-(2-ethyl-hexyloxy)-2-hydroxy]-phenyl}-6-(4-methoxyphenyl)-1,3,5-triazin und die zweite UV-Filtersubstanz in Komponente (a) Phenylen-1,4-bis-(2-benzimidazyl)-3,3'-5,5'-tetrasulfonsäure-bis-natriumsalz ist, und
(b) mindestens einen Füllstoff, ausgewählt aus der Gruppe von synthetischen organischen Polymeren enthält.

3. Zubereitung nach Anspruch 1 oder 2, **dadurch gekennzeichnet, dass** als weitere UV-Filtersubstanz(en) das oder die unsymmetrisch substituierten Triazin(e) ausgewählt werden aus der Gruppe bestehend aus:
Dioctylbutylamidotriazon,
2,4-Bis-{[4-(3-sulfonato)-2-hydroxy-propyloxy)-2-hydroxy]-phenyl}-6-(4-methoxyphenyl)-1,3,5-triazin Natriumsalz,
2,4-Bis-{[4-(3-(2-propyloxy)-2-hydroxy-propyloxy)-2-hydroxy]-phenyl}-6-(4-methoxyphenyl)-1,3,5-triazin,
2,4-Bis-{[4-(2-ethyl-hexyloxy)-2-hydroxy]-phenyl}-6-[4-(2-methoxyethyl-carboxyl)-phenylamino]-1,3,5-triazin,
2,4-Bis-{[4-(3-(2-propyloxy)-2-hydroxy-propyloxy)-2-hydroxy]-phenyl}-6-[4-(ethylcarboxyl)-phenylamino]-1,3,5-triazin,
2,4-Bis-{[4-(2-ethyl-hexyloxy)-2-hydroxy]-phenyl}-6-(1-methyl-pyrrol-2-yl)-1,3,5-triazin,
2,9-Bis-{[4-tris(trimethylsiloxy-silylpropyloxy)-2-hydroxy]-phenyl}-6-(4-methoxyphenyl)-1,3,5-triazin,
2,4-Bis-{[4-(2-methylpropenyloxy)-2-hydroxy]-phenyl}-6-(4-methoxyphenyl)-1,3,5-triazin und
2,4-Bis-{[4-(1',1',1',3',5',5',5'-Heptamethylsiloxy-2-methyl-propyloxy)-2-hydroxy]-phenyl}-6-(4-methoxyphenyl) -1, 3, 5-triazin.

4. Zubereitung nach einem der vorhergehenden Ansprüche, **dadurch gekennzeichnet, dass** der mindestens eine Füllstoff ausgewählt ist aus Nylon und Polymethylmethacrylate.

5. Zubereitung nach einem der vorhergehenden Ansprüche **dadurch gekennzeichnet, dass** sie als UV Filter 2,4-Bis-{[4-(2-ethyl-hexyloxy)-2-hydroxy]-phenyl}-6-(4-methoxyphenyl)-1,3,5-triazin, Phenylen-1,4-bis-(2-benzimidazyl)-3,3'-5,5'-tetrasulfon-säure-bis-natriumsalz, und 2,2'-Methylen-bis-(6-(2H-benzotriazol-2-yl)-4-(1,1,3,3-tetramethylbutyl)-phenol) in Kombination mit Talkum oder Kaolin als Füllstoff enthält.

6. Zubereitung nach einem der vorhergehenden Ansprüche, welche ein samtiges oder seidiges Hautgefühl hervorruft oder verstärkt, **dadurch gekennzeichnet, dass** sie ferner mindestens ein in Wasser nicht quellbares Schichtsilikat und/oder sphärisches Siliziumdioxid als weitere(n) Füllstoff(e) der Komponente (b) enthält.

7. Verwendung von Zubereitungen nach Anspruch 1 oder einem der Ansprüche 3 bis 6, soweit auf Anspruch 1 zurückbezogen, zum Schutz von Haut und Haar gegen die schädlichen Wirkungen von UV-A- und UV-B-Strahlung, insbesondere Sonnenstrahlung, **dadurch gekennzeichnet, dass** die Zubereitung zur Dekoration von Haut, Lippe und/oder Haar dient.

8. Verwendung von Zubereitungen nach Anspruch 2 oder einem der Ansprüche 3 bis 6, soweit auf Anspruch 2 zurückbezogen, zur Herstellung eines Mittels zum Schutz von Haut und Haar gegen die schädlichen Wirkungen von UV-A- und UV-B-Strahlung, insbesondere Sonnenstrahlung.

9. Verwendung nach Anspruch 8, **dadurch gekennzeichnet, dass** das Mittel zum Schutz der Haut vor vorzeitiger Alterung, insbesondere der Faltenbildung und Elastizitätsverlust verwendet wird.

## Claims

1. Cosmetic preparation, **characterized in that** it comprises
(a) at least 2 UV filter substances, where the first UV filter substance is 2,4-bis{[4-(2-ethylhexyloxy)-2-hydroxy]phenyl}-6-(4-methoxyphenyl)-1,3,5-triazine and the second UV filter substance in component (a) is phenylene-1,4-bis(2-benzimidazyl)-3,3',5,5'-tetrasulfonic acid disodium salt, and
(b) at least one filler selected from the group of synthetic organic polymers.

2. Dermatological preparation, **characterized in that** it comprises
(a) at least 2 UV filter substances, where the first UV filter substance is 2,4-bis{[4-(2-ethylhexyloxy)-2-hydroxy]phenyl}-6-(4-methoxyphenyl)-1,3,5-triazine and the second UV filter substance in component (a) is phenylene-1,4-bis(2-benzimidazyl)-3,3',5,5'-tetrasulfonic acid disodium salt, and
(b) at least one filler selected from the group of synthetic organic polymers.

3. Preparation according to Claim 1 or 2, **characterized in that**, as further UV filter substance(s), the asymmetrically substituted triazine(s) is (are) selected from the group consisting of:
dioctylbutylamidotriazone,
2,4-bis{[4-(3-sulfonato)-2-hydroxypropoxy)-2-hydroxy]phenyl}-6-(4-methoxyphenyl)-1,3,5-triazine sodium salt,
2,4-bis{[4-(3-(2-propoxy)-2-hydroxypropoxy)-2-hydroxy]phenyl}-6-(4-methoxyphenyl)-1,3,5-triazine,
2,4-bis{[4-(2-ethylhexyloxy)-2-hydroxy]phenyl}-6-[4-(2-methoxyethylcarboxy)phenylamino]-1,3,5-triazine,
2,4-bis{[4-(3-(2-propoxy)-2-hydroxypropoxy)-2-hydroxy]phenyl}-6-[4-(ethylcarboxy)-phenylamino]-1,3,5-triazine,
2,4-bis{[4-(2-ethylhexyloxy)-2-hydroxy]phenyl}-6-(1-methylpyrrol-2-yl)-1,3,5-triazine,
2,4-bis{[4-tris(trimethylsiloxysilylpropoxy)-2-hydroxy]phenyl}-6-(4-methoxyphenyl)-1,3,5-triazine,
2,4-bis{(4-(2-methylpropenyloxy)-2-hydroxy]phenyl}-6-(4-methoxyphenyl)-1,3,5-triazine and
2,4-bis{[4-(1',1',1',3',5',5',5'-heptamethylsiloxy-2-methylpropoxy)-2-hydroxy]phenyl}-6-(4-methoxyphenyl)-1,3,5-triazine.

4. Preparation according to one of the preceding claims, **characterized in that** the at least one filler is selected from nylon and polymethyl methacrylate.

5. Preparation according to one of the preceding claims, **characterized in that** it comprises, as UV filters, 2,4-bis{[4-(2-ethylhexyloxy)-2-hydroxy]phenyl}-6-(4-methoxyphenyl)-1,3,5-triazine, phenylene-1,4-bis(2-benzimidazyl)-3,3',5,5'-tetrasulfonic acid disodium salt and 2,2'-methylenebis(6-(2H-benzotriazol-2-yl)-4-(1,1,3,3-tetramethylbutyl)phenol in combination with talk or kaolin as filler.

6. Preparation according to one of the preceding claims, which causes or enhances a velvety or silky skin feel, **characterized in that** it furthermore comprises at least one non-water-swellable phyllosilicate and/or spherical silicon dioxide as further filler(s) of component (b).

7. Use of preparations according to Claim 1 or one of Claims 3 to 6, in so far as dependent on Claim 1, for protecting skin and hair against the damaging effects of UV-A and UV-B radiation, in particular solar radiation, **characterized in that** the preparation serves for the decoration of skin, lips and/or hair.

8. Use of preparations according to Claim 2 or one of Claims 3 or 6, in so far as dependent on Claim 2, for the preparation of a composition for protecting skin and hair against the damaging effects of UV-A and UV-B radiation, in particular solar radiation.

9. Use according to Claim 8, **characterized in that** the composition is used to protect the skin against premature ageing, in particular wrinkling and loss of elasticity.

## Revendications

1. Composition cosmétique, **caractérisée en ce qu'**elle comprend :
(a) au moins 2 filtres UV, le premier filtre UV étant la 2,4-bis-{[4-(2-éthylhexyloxy)-2-hydroxy]-phényl}-6-(4-méthoxyphényl)-1,3,5-triazine et le deuxième filtre UV dans le composant (a) étant le sel disodique d'acide phénylène-1,4-bis-(2-benzimidazyl)-3,3'-5,5'-tétrasulfonique, et
(b) au moins une charge, choisie dans le groupe constitué par les polymères organiques synthétiques.

2. Composition dermatologique, **caractérisée en ce qu'**elle comprend :
(a) au moins 2 filtres UV, le premier filtre UV étant la 2,4-bis-{[4-(2-éthylhexyloxy)-2-hydroxy]-phényl}-6-(4-méthoxyphényl)-1,3,5-triazine et le deuxième filtre UV dans le composant (a) étant le sel disodique d'acide phénylène-1,4-bis-(2-benzimidazyl)-3,3'-5,5'-tétrasulfonique, et
(b) au moins une charge, choisie dans le groupe constitué par les polymères organiques synthétiques.

3. Composition selon la revendication 1 ou 2, **caractérisée en ce que** l'on choisit, comme autre(s) filtre(s) UV, la ou les triazines asymétriquement substituées dans le groupe constitué par:
la dioctylbutylamidotriazone,
le sel sodique de 2,4-bis-{[4-(3-sulfonato)-2-hydroxy-propyloxy)-2-hydroxy]-phényl}-6-(4-méthoxyphényl)-1,3,5-triazine,
la 2,4-bis-{[4-(3-(2-propyloxy)-2-hydroxy-propyloxy)-2-hydroxy]-phényl}-6-(4-méthoxyphényl)-1,3,5-triazine,
la 2,4-bis-{[4-(2-éthylhexyloxy)-2-hydroxy]-phényl}-6-[4-(2-méthoxyéthyl-carboxyl)-phénylamino]-1,3,5-triazine,
la 2,4-bis-{[4-(3-(2-propyloxy)-2-hydroxy-propyloxy)-2-hydroxy]-phényl}-6-[4-(éthylcarboxyl)-phénylamino]-1,3,5-triazine,
la 2,4-bis-{[4-(2-éthylhexyloxy)-2-hydroxy]-phényl}-6-(1-méthyl-pyrrol-2-yl]-1,3,5-triazine,
la 2,4-bis-{[4-tris(triméthylsiloxy-silylpropyloxy)-2-hydroxy]-phényl}-6-(4-méthoxyphényl)-1,3,5-triazine,
la 2,4-bis-{[4-(2-méthylpropényloxy)-2-hydroxy]-phényl}-6-(4-méthoxyphényl)-1,3,5-triazine, et
la 2,4-bis-{[4-(1',1',1',3',5',5',5'-heptaméthylsiloxy-2-méthylpropyloxy)-2-hydroxy]-phényl}-6-(4-méthoxyphényl)-1,3,5-triazine.

4. Composition selon l'une des revendications précédentes, **caractérisée en ce que** la au moins une charge est choisie parmi le nylon et le polyméthacrylate de méthyle.

5. Composition selon l'une des revendications précédentes, **caractérisée en ce qu'**elle comprend, comme filtre UV, la 2,4-bis-{[4-(2-éthylhexyloxy)-2-hydroxy]-phényl}-6-(4-méthoxyphényl)-1,3,5-triazine, le sel disodique d'acide phénylène-1,4-bis-(2-benzimidazyl)-3,3'-5,5'-tétrasulfonique, et le 2,2'-méthylène-bis-(6-(2H-benzotriazol-2-yl)-4-(1,1,3,3-tétraméthylbutyl)-phénol) en combinaison avec du talc ou du kaolin comme charge.

6. Composition selon l'une des revendications précédentes, qui provoque ou accentue la sensation de peau de velours ou de peau soyeuse, **caractérisée en ce qu'**elle comprend en outre au moins un silicate lamellaire non gonflable dans l'eau et/ou du dioxyde de silicium sphérique comme autre(s) charge(s) du composant (b).

7. Utilisation des compositions selon la revendication 1 ou l'une des revendications 3 à 6, dans la mesure où elle se rapporte à la revendication 1, pour la protection de la peau et des cheveux contre les actions nocives du rayonnement UV-A et UV-B, en particulier du rayonnement solaire, **caractérisée en ce que** la composition sert à la décoration de la peau, des lèvres et/ou des cheveux.

8. Utilisation des compositions selon la revendication 2 ou l'une des revendications 3 à 6, dans la mesure où elle se rapporte à la revendication 2, pour la fabrication d'un agent pour la protection de la peau et des cheveux contre les actions nocives du rayonnement UV-A et UV-B, en particulier du rayonnement solaire.

9. Utilisation selon la revendication 8, **caractérisée en ce que** l'agent est utilisé pour la protection de la peau contre le vieillissement prématuré, en particulier la formation de rides et la perte d'élasticité.
